# EUROPEAN PATENT SPECIFICATION

(11) **EP 2 491 143 B1**
(45) Date of publication and mention of the grant of the patent: **13.12.2017**
(21) Application number: 10825619.9
(22) Date of filing: 20.10.2010
(51) Int. Cl.: A61K 35/12, A61K 38/17, G01N 33/564

(54) **METHODS AND COMPOSITIONS FOR DIAGNOSIS AND TREATMENT OF GENETIC AND RETINAL DISEASE**
VERFAHREN UND ZUSAMMENSETZUNGEN ZUR DIAGNOSE UND BEHANDLUNG VON GENETISCHEN ODER RETINALERKRANKUNGEN
MÉTHODES ET COMPOSITIONS UTILISÉES POUR LE DIAGNOSTIC ET LE TRAITEMENT D'UNE RÉTINOPATHIE GÉNÉTIQUE

(30) Priority: 21.10.2009 US 253541 P
(43) Date of publication of application: 29.08.2012
(73) Proprietor: Retinal Solutions LLC, Keene, NH 03431 (US)
(72) Inventor: DRENSER, Kimberly, Bloomfield, MI 48302 (US)
(74) Representative: Plougmann Vingtoft a/s
(86) International application number: PCT/US2010/053422
(87) International publication number: WO 2011/050096

(56) References cited:
- WO-A2-01/81634
- WO-A2-02/092635
- WO-A2-03/004045
- WO-A2-03/005034
- WO-A2-2008/074839
- JP-A- 2000 093 186
- US-A1- 2004 072 266

## Description

### CROSS-REFERENCE TO RELATED APPLICATIONS

This application claims priority of U.S. Provisional Patent Application Ser. No. 61/253,541 filed Oct. 21,2009.

### FIELD OF THE INVENTION

The subject invention relates generally to methods and therapies for the treatment of ocular diseases due to acquired retinal or vascular degeneration or genetic abnormality. More specifically, the subject invention relates to treatments and therapeutics to promote vascular and neuronal growth or differentiation in the retinal bed. Most specifically, the subject disclosure relates to methods and compositions for treatment of Norrie disease, familial exudative vitreoretinopathy, retinopathy of prematurity, or other related genetic and acquired vitreoretinal and vascular developmental diseases.

### BACKGROUND OF THE INVENTION

Proper vascular modeling in the retina is essential for ocular development and visual acuity. Abnormal vessel growth during development or in adulthood produces several diseases such as retinopathy of prematurity, diabetic retinopathy, and age-related macular degeneration. Normal retinal development occurs through vessels forming at the optic nerve head and spreading over the retina to form a dense network. Connolly, S E, et al., Microvasc Res, 1988; 36:275-290; Provis, J M, Prog Retin Eye Res, 2001; 20:799-821; Fruttiger, M, Invest Ophthalmol Vis Sci, 2002; 43:522-527. Development proceeds through formation of primary vessels along the surface of the developing retina from which divergent vessels begin to extend into the capillary beds that form the outer and inner plexiform layers of the retina. Connelly, 1988; Provis, 2001, Fruttiger, 2002. Vascular development is mediated by a series of growth factors that direct formation and extension of new vessels. Retinal development is unique in the concentration and types of signaling mediators employed to promote angiogenic sprouting from the primary vascular network and the formation of the final capillary architecture. Ohlmann, A, et al., J Neurosci, 2005; 25:1701-1710.

One factor involved in formation of primary retinal vascular and retinal capillaries is the transmembrane receptor frizzled-4 (Fzd-4). Mutations in the gene encoding the Fzd-4 receptor are observed in patients diagnosed with autosomal dominant Familial Exudative Retinopathy (AdFEVR), Toomes C, Downey L, GeneReviews, NCBI, 2008, and retinopathy of prematurity (ROP). MacDonald, M L, Clin Genet., 2005; 67:363-6. In both diseases, the patient is born with enlarged and tortuous retinal vessels and an area of avascular peripheral retina. Additionally, varying degrees of subretinal exudation, vitreoretinal traction, and abnormal extraretinal vessels/neovascularization may occur.

Fzd-4 is a 537 amino acid, seven transmembrane receptor that functions via three signaling pathways. Proper Fzd-4 ligand binding and signal transduction are required for normal retinal vascular development. Several ligands are known to interact with Fzd-4 to produce signaling events. These include the Wnt ligands (Wnt-3a, Wnt-8, and Wnt-5a) and the non-Wnt ligand Norrin.

Norrin is encoded by the NDP gene present on chromosome X at position 11.4. The importance of this gene product is highlighted by observations that inactivating mutations lead to Norrie disease which is characterized by ocular and cochlear vascular defects. Rhem, H L, et al., J Neurosci, 2002; 22:4286-4292; Black, G C, et al., Hum Mol Genet, 1999; 8:2031-2035. Silencing of the NDP gene produces incomplete regression of the primary hyaloid system and abnormal retinal maturation.

The 131 amino acid Norrin protein is secreted into the extracellular space. Meitinger, T, et al., Nat Genet, 1993; 5:376-380; Berger, W, et al., Hum Mol Genet, 1996; 5:51-59. Two primary domains define the general Norrin protein structure: a signal peptide directs localization of the molecule; and a cysteine-knot motif provides the tertiary confirmation required for receptor binding and activation of signal transduction.

The importance of the cysteine knot-motif is highlighted by computer modeling that demonstrates the requirement of disulfide bonds between the cysteine residues in forming the structural confirmation of Norrin. Mutation(s) of the cysteine residues reduces the affinity of Norrin for its receptor and prevents activation of subsequent signaling pathways. Mutations in these residues also result in severe retinal dysgenesis and Norrie disease. However, mutations in regions other than the cysteine knot-motif produce incomplete protein folding and result in FEVR and related vitreoretinopathies (Retinopathy of Prematurity, persistent fetal vasculature).

Norrin is a ligand for the Fzd-4. Norrin binds Fzd-4 with nanomolar affinity and stimulates a Wnt receptor:β-catenin signal transduction pathway that regulates retinal development and is necessary for regression of hyaloid vessels in the eye. Xu, Q, et al., Cell, 2004; 116:883-895; Clevers, H, Curr Biol, 2004; 14:R436-437; Nichrs, C, Dev Cell, 2004; 6:453-454. Norrin interaction with Fzd-4 is dependent on the cell surface receptor lipoprotein receptor related protein 5 (LRP5). Xu, 2004.

Fzd-4 signaling is mediated by three independent signal transduction pathways each of which is believed to be activated by binding of any of the Fzd-4 ligands. These pathways include the canonical Wnt/β-Catenin pathway, the planar cell polarity pathway, and the Wnt-Ca²⁺ pathway. Signaling is initiated by ligand binding to Fzd-4 alone or along with its co-receptor LRP5.

The most recognized and studied Fzd-4 signaling mechanism is the Wnt/ β-Catenin pathway. Ligand binding inactivates glycogen synthase kinase (GSK) 313 and Axin resulting in dephosphorylation of β-catenin and its translocation to the nucleus. The inactivation of these proteins stabilizes β -catenin, which subsequently accumulates in the cell nucleus and activates the transcription factor and lymphoid enhancer-binding factor (TCF/LEF-1) family of DNA binding proteins regulating transduction of target genes crucial in the G1-S-phase transition, encoding proteins such as cyclin D1, VEGF, or c-Myc. Willert K, and Nusse R, Curr Opin Genet Dev, 1998; 8:95-102. These pathways promote stimulation and proliferation of retinal stem cells. Inoue, T, et al., Stem Cells, 2006; 24:95-104.

The planar cell polarity pathway is activated by any ligand binding to Fzd-4. Unlike the canonical pathway, the planar cell polarity pathway does not require association of Fzd-4 with LRP5 possibly promoting differential regulation and expression of this pathway based on the presence or activity of LRP5. This pathway is propagated by activation of dischevelled protein (Dsh) which leads to activation of Rho or Rac promoting expression of alternative signal transduction pathways. The planar cell polarity pathway mediates cytoskeletal organization and cell migration.

Finally, the Wnt- Ca²⁺ pathway produces release of intracellular Ca²⁺. As for other signaling systems the increase in Ca²⁺ concentration in the cytoplasm leads to activation of calcium-calmodulin kinase 2 (CamKII) and protein kinase C (PKC). This pathway controls cell adhesion and cell movement during gastrulation.

Abnormalities in the Fzd-4 and LRP5 receptors result in the phenotypically similar conditions FEVR, ROP, and possibly Norrie disease. Robitaille, J, et al., Nature Genet, 2002; 32:326-330; Kondo, H, et al., Br J Opthalmol, 2003; 87:1291-1295; Toomes, C, et al., Am J Hum Genet, 2004; 74:721-730. The close association between the phenotypes produced by Norrin mutations and mutations in the Fzd-4 and LRP5 receptors bolsters the hypothesis that these molecules form a functional signaling group. Planutis, K, et al., BMC Cell Biology, 2007; 8:12.
WO 03/005034 discloses mutations in selected genes, such as the Frizzled-4 gene ("fzd4"), which are causative agents in hereditary human visual disorders, such as familial exudative vitreoretinopathy ("FEVR").
WO 03/004045 discloses mutations in Wnt receptor genes, such as FZD4, associated with hereditary human visual disorders, such as familial exudative vitreoretinopathy ("FEVR").
US 20040072266 discloses method of modifying cell motility via compounds and polypeptides that interact with a Wnt pathway.

While some defects in the Fzd-4 gene have been correlated to incomplete or immature vascularization and disease, these studies do not explain the full extent and occurrence of the disease. Further, therapies presently available for Norrie disease, FEVR, ROP, or other retinal diseases are only modestly effective. Thus, there exists a need for improved methods of identification and diagnosis of retinal diseases. There also exists a need for therapeutics and methods of treatment for vitreoretinal disease and vascular disease in the retina.

### SUMMARY OF THE INVENTION

In here is disclosed methods and materials for identifying and diagnosing disease by determining the presence or absence of a mutation in the gene encoding Fzd-4 in a subject. Also disclosed are methods for altering or maintaining physiological activity that involve administering a compound to a subject and measuring at least one parameter indicative of physiological activity in the subject. The physiological activity is optionally vascularization, cell proliferation, cellular interaction, neuroprotection, growth, vascular regression, b-wave response, cell viability, or substantial oscillatory potential.

The disclosed process optionally includes administering a cell to the subject. The cell is optionally transfected with a nucleotide sequence encoding an Fzd-4 compound, and administering the transfected cell to the subject. The cell is optionally a stem cell.

Numerous methods of administration are operable in the inventive methods illustratively including: systemic administration, local administration, injection, topical administration, intraocular, and iontophoretic delivery.

The disclosed methods illustratively include administration of the compound to a subject. A subject is illustratively a mammal, human, cow, horse, sheep, pig, goat, chicken, cat, dog, mouse, guinea pig, hamster, rabbit, rat, and a cell.

The disclosed method is preferably used to treat a subject with a pathological condition of the retina or is at risk of developing a pathological condition or the retina. The pathological condition is preferably caused by lacking a protein or a mutant protein, or by an acquired degeneration or disease of the retina requiring proliferation of progenitor cells. The pathological condition is optionally vitreoretinopathy, retinopathy of prematurity, familial exudative vitreoretinopathy, Norrie disease, persistent fetal vasculature, and macular degeneration.

The compound of the subject inventive methods is optionally recombinant. The compound further optionally has a marker. The marker is optionally green fluorescent protein, luciferase, and/or β -galactosidase.
In a first aspect, the invention relates to a process for diagnosing a disease or a susceptibility to a disease related to expression, conformation, or activity of the Fzd-4 polypeptide in a subject comprising:
- providing a sample from said subject;
- determining in the sample the presence or absence of a substitution mutation in the nucleotide sequence encoding Fzd-4 in the genome at nucleotide 349 and/or 542 of SEQ ID NO: 1; and
- diagnosing the presence or absence of said disease or susceptibility to said disease in said subject or descendents of said subject;
wherein presence of the substitution mutation in the nucleotide sequence encoding Fzd-4 in the genome at nucleotide 349 and/or 542 of SEQ ID NO: 1 indicates the presence or susceptibility for a disease related to expression, conformation, or activity of the Fzd-4 polypeptide in the subject;
wherein the substitution mutation at nucleotide 349 and/or 542 of SEQ ID NO: 1 cause C117R and/or C181Y mutation in the Fzd-4 polypeptide;
wherein the disease is familial exudative vitreoretinopathy (FEVR).
In another aspect, the invention relates to an Fzd-4 for use as a medicament in the treatment of familial exudative vitreoretinopathy (FEVR) in a subject having a pathological condition of the retina or at risk of developing a pathological condition of the retina resulting from substitution mutation at nucleotide 349 and/or 542 of the nucleotide sequence genome encoding Fzd-4 polypeptide;
wherein the substitution mutation at nucleotide 349 and/or 542 of SEQ ID NO: 1 cause C117R and/or C181Y mutation in the Fzd-4 polypeptide.

### BRIEF DESCRIPTION OF THE DRAWINGS

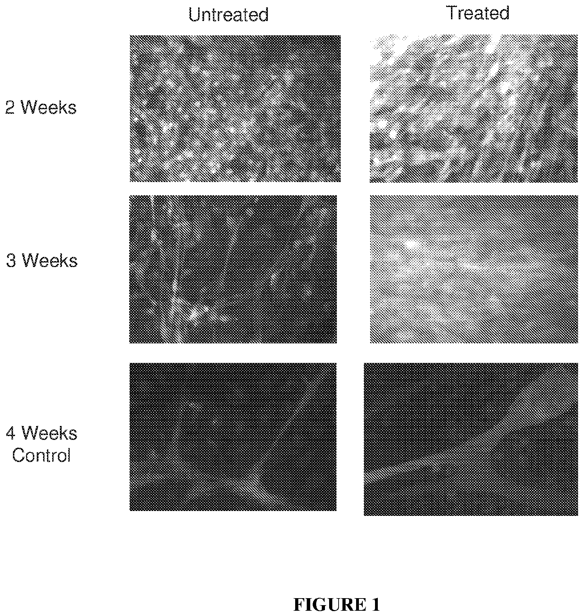

FIG. 1 depicts improved vascular development in the presence of cells expressing Fzd-4.

### DETAILED DESCRIPTION OF THE PREFERRED EMBODIMENTS

It is to be understood that the present invention is not limited to particular embodiments described, as such may, of course, vary. It is also to be understood that the terminology used herein is for the purpose of describing particular embodiments only, and is not intended to be limiting.

The present invention provides a process for diagnosing the presence of or susceptibility to a disease related to the Fzd-4 protein. The inventive process identifies the presence or absence of one or more mutations in the gene sequence encoding Fzd-4. Particular preferred mutational sites are nucleotides 349 and 542 of SEQ ID NO: 1. Identification of the presence or absence of a mutation in Fzd-4 or the gene encoding Fzd-4 allows diagnosis of the presence or susceptibility to disease.
Thus, in a first aspect, the invention relates to a process for diagnosing a disease or a susceptibility to a disease related to expression, conformation, or activity of the Fzd-4 polypeptide in a subject comprising:
- providing a sample from said subject;
- determining in the sample the presence or absence of a substitution mutation in the nucleotide sequence encoding Fzd-4 in the genome at nucleotide 349 and/or 542 of SEQ ID NO: 1; and
- diagnosing the presence or absence of said disease or susceptibility to said disease in said subject or descendents of said subject;
wherein presence of the substitution mutation in the nucleotide sequence encoding Fzd-4 in the genome at nucleotide 349 and/or 542 of SEQ ID NO: 1 indicates the presence or susceptibility for a disease related to expression, conformation, or activity of the Fzd-4 polypeptide in the subject;
wherein the substitution mutation at nucleotide 349 and/or 542 of SEQ ID NO: 1 cause C117R and/or C181Y mutation in the Fzd-4 polypeptide;
wherein the disease is familial exudative vitreoretinopathy (FEVR).

A process for diagnosis and treatment of degenerative or inherited ocular diseases is provided. Thus, the subject disclosure has utility for the identification, prevention, reversal, or treatment of vitreoretinal and vascular disease or cancer.

As used herein, the term "subject" illustratively includes a mammal, human, cow, horse, sheep, pig, goat, chicken, cat, dog, mouse, guinea pig, hamster, rabbit, rat, a cell, tissue, organ, organ system, or combinations thereof. It is appreciated that the term subject is optionally a patient.

As used herein, the terms "pathological condition" or "disease" illustratively include vitreoretinopathy, retinopathy of prematurity (retrolental fibroplasias), Familial Exudative Vitreoretinopathy (FEVR), Norrie disease, Persistent Fetal Vasculature Syndrome (persistent hyperplastic primary vitreous), Coats disease, Macular Degeneration, Macular Dystrophy, inherited dystrophy, cancer, growth of abnormal cells, Osteoporosis-Pseudoglioma Syndrome, Non-Exudative Age Related Macular Degeneration (ARMD), Exudative Age Related Macular Degeneration (ARMD), Choroidal Neovascularization, Diabetic Retinopathy, Retinal Vein Occlusion, Retinal Artery Occlusion, Acute Macular Neuroretinopathy, Central Serous Chorioretinopathy, Cystoid Macular Edema, Diabetic Macular Edema, Acute Multifocal Placoid Pigment Epitheliopathy, Behcet's Disease, Birdshot Retinochoroidopathy, Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpignous Choroiditis, Subretinal Fibrosis and Uveitis Syndrome, Vogt-Koyanagi-Harada Syndrome, Parafoveal Telangiectasis, Papillophlebitis, Frosted Branch Angitis, Sickle Cell Retinopathy and other Hemoglobinopathies, Angioid Streaks, Sympathetic Ophthalmia, Uveitic Retinal Disease, Retinal Detachment, Trauma, Laser, PDT, Photocoagulation, Hypoperfusion During Surgery, Radiation Retinopathy, Bone Marrow Transplant Retinopathy, Proliferative Vitreal Retinopathy and Epiretinal Membranes, Proliferative Diabetic Retinopathy, Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associated with HIV Infection, Uveitic Disease associated with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis, Systemic Disorders with Associated Retinal Dystrophies, Congenital Stationary Night Blindness, Cone Dystrophies, Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum, Osler Weber syndrome, Retinal Detachment, Macular Hole, Giant Retinal Tear, Retinal Disease Associated with Tumors, Solid Tumors, Tumor Metastasis, Benign Tumors, for example, hemangiomas, neurofibromas, trachomas, and pyogenic granulomas, Congenital Hypertrophy of the RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, Intraocular Lymphoid Tumors, Punctate Inner Choroidopathy, Acute Posterior Multifocal Placoid Pigment Epitheliopathy, Myopic Retinal Degeneration, Acute Retinal Pigment Epithelitis, Ocular inflammatory and immune disorders, ocular vascular malfunctions, Corneal Graft Rejection, Neovascular Glaucoma, and other diseases of ocular vascular development.

Signaling through Fzd-4 produces increased angiogenesis and is found in endothelial tumors. Aberrant signaling through Fzd-4 is strongly associated with colorectal cancer. Similarly, increased expression of Fzd-4 or Wnt signaling correlates with acute myeloid leukemia (AML). For example, mutations in Flt3 lead to increased Fzd-4 mRNA and protein levels in patients with AML. This correlates with increased β-catenin signaling and onset of AML. Thus, Fzd-4 has a role in onset of AML and other cancers. In a preferred embodiment, the inventive method is used to determine the presence or absence of one or more mutations in Fzd-4 that increase the affinity for a ligand, increase the level of signaling, or increase the mRNA or protein levels of Fzd-4 that correlate with tumorogenesis. Preferably, mutations in the Fzd-4 gene lead to increased expression. Mutations are preferably in regions that regulate transcription such as in upstream or downstream promoters, enhancers, polymerase and accessory protein binding sites, binding sites for DNA structural accessory proteins, or other regions that regulate the expression level of Fzd-4. The genomic sequence of Fzd-4 in a subject and locations of mutational sites are illustratively found in Genbank accession number NC--000011. Illustratively, STAT5b binding to tandem promoter elements in Fzd-4 regulates gene transcription. These sites and other STAT5b binding sights are illustrated in Vidal, O M, et al., Mol Endrocrin, 2007; 21: 293-311. Determining the presence or absence of mutations in these regions illustratively is used in the subject disclosure to diagnose a disease or susceptibility to a disease such as cancer, specifically, AML.

Mutations in Fzd-4 produce reduced angiogenesis and correlate with decreased susceptibility to solid tumor cancers. For example, vascular endothelial cells that express Fzd-4 undergo proliferation and sprouting angiogenesis upon exposure to Fzd-4 ligands such as Wnt2. Mutations, including those described in this specification, regulate the activity or ligand binding affinity of Fzd-4 protein. Without being bound to a particular theory, illustratively, the Cys117Arg mutation alters the secondary structure in the ligand binding domain reducing affinity for Norrin or Wnt ligands. This reduction in ligand affinity reduces Fzd-4 signaling activity and angiogenic propensity of carrier cells. Thus, identification of the Cys117Arg mutation, as well as other mutations described herein in Fzd-4, LRP5, LRP6, or Norrin are operable for determining the susceptibility to cancer or diagnosis of cancer or tumor types.

Mutations in LRP5 correlate with bone density in subjects. Loss of function mutations in LRP5 produce reduced bone density and promote osteoporosis pseudoglioma syndrome whereas gain of function mutations produce increased bone density. In a preferred part of the disclosure, the process analyzes the association of LRP5 with Fzd-4 to form a co-receptor for Wnt ligands and Norrin by screening for mutations that alter the signaling characteristics, protein-protein interactions such as co-receptor association, receptor dimerization, and co-receptor ligand association to diagnose or determine the susceptibility to a disease such as osteoporosis pseudoglioma syndrome or other bone density abnormalities. LRP5 mutations operable in the present invention in addition to mutations in Fzd-4 in the process of the present invention and their effects are reviewed by Balemans, W, and Van Hul, W, Endocrinology, 2007; 148:2622-2629.

As used herein, the term "physiological activity" illustratively includes vascularization, cell proliferation, cellular interaction, cellular differentiation, reduction in apoptosis, reduction in necrosis, neuroprotection, growth, vascular regression, CAMKII phosphorylation, protein kinase C (PKC) activation, protein kinase A activation, activation of the MAPK pathway (illustratively, MAPK8 or JNK), downstream gene activation, activation of the β -catenin pathway, activation of the Wnt/ β -catenin pathway, activation of the planar cell polarity pathway, cellular viability, proteolytic activity, phosphorylation, dephosphorylation, receptor activation, receptor inactivation, other activities illustratively describe by Lin, S, et al., Molecular Vision 2009; 15:26-37, or combinations thereof.

An inventive process includes diagnosing or identifying a disease or susceptibility to a disease related to the expression or activity of the Fzd-4 polypeptide in a subject. In a preferred embodiment the inventive process includes determining the presence or absence of a mutation in the nucleotide sequence encoding the Fzd-4 protein. The nucleotide sequence is preferably the genetic sequence, an mRNA sequence, cDNA sequence, or other sequence associated with the Fzd-4 protein. Preferably, the Fzd-4 sequence is the gene sequence as found in the DNA of a subject. Optionally, the Fzd-4 sequence is an mRNA sequence. An example of an mRNA sequence of Fzd-4 is found at GenBank accession no. NM_012193.2. An alternative example of a Fzd-4 nucleotide sequence is SEQ ID NO: 1. The mutations of the Fzd-4 gene as further described in this specification preferably refer to the nucleotide numbering of SEQ ID NO: 1.

The disclosed process preferably determines the presence or absence of a nucleotide substitution in the Fzd-4 gene at nucleotide 349, 542, 1513, 97, or 502 of SEQ ID NO: 1. Any combination of substitutions at nucleotide 349, 542, 1513, 97, or 502 of SEQ ID NO: 1 are operable herein. In a most preferred embodiment a substitution at nucleotide 349 or 542 is determined. Optionally, an inventive method determines the presence or absence of more than one nucleotide substitution. Illustratively, the inventive method screens for a substitution at nucleotide positions 349, 97, and 502. It is appreciated that other combinations of mutations are similarly operable. The presence of more than one substitution provides additional information useful for the diagnosis of the presence of or susceptibility to a disease.

As used herein the term "susceptibility" includes a likelihood of developing a disease, being a carrier for a disease, or likelihood of genetic transmission of the disease to progeny.

An inventive process preferably determines the presence or absence of a mutation in a nucleotide sequence of the Fzd-4 gene in a subject that translates to an amino acid substitution, chain termination (nonsense mutation), or coding frame shift mutation in the Fzd-4 protein. Examples of mutations operable in the subject disclosure include Cys117Arg, Cys181Tyr, Gln505X, Pro33Ser, Pro168Ser, Met105Val, Met105Thr, Arg417Gln, Gly488Asp, Trp319X, His69Tyr, Thr445Pro, and Gly492Arg. In a more preferred embodiment a mutation producing a mutation at Cysl117 or Cys181 is detected. Most preferably, a mutation producing the amino acid substitution Cys117Arg or Cys181Tyr is detected. Other mutations such as those described by Nesta T, et al., Physiology, 2006; 21: 181-188; Gal, A, et al., Invest Ophthalmol Vis Sci, 2004; 45: Abst. 4734; and Kondo, H, et al., Br J Ophthalmol, 2003; 87: 1291-1295, are similarly operable in the present disclosure.

In one embodiment more than one mutation producing an amino acid substitution is detected. Preferably, two, three, or four mutations are detected either simultaneously or sequentially. It is appreciated that more than four mutations are optionally detected. Preferably, mutations substituting Cys117 and Cys181 are detected. Optionally, Cys117, Pro33, and Pro168 substitutions are detected. It is appreciated that any combination of mutations is detectable and used to diagnose or predict disease.

In addition to detection of mutations in the oligonucleotide sequence for Fzd-4, the disclosed process analyzes the sequence for LRP5 and Norrin. Analyses of the presence or absence of a mutation in the gene, mRNA, or protein of LRP5 and Norrin are optionally performed simultaneous with or sequential to analyses of the Fzd-4 sequence. Mutations in LRP5 and Norrin that segregate with disease are reviewed in Nestor, T, et al., Physiology, 2006; 21:181-188. It is appreciated that other mutations that segregate with disease in either LRP5 or Norrin are similarly operable for the diagnosis of or susceptibility to disease in the present disclosure. In one embodiment a subject is screened for mutations in both Fzd-4 and LRP5. The presence of multiple mutations in the genes for multiple signaling molecules provides additional or confirmatory evidence as to the presence of or susceptibility to disease. Further, detection of mutations in one or more genes is useful for identifying the extent of or specific type of disease. For example, LRP5 is known to participate only in the canonical signaling pathway. In vitro studies also found some role for LRP5 in the Wnt/ β -catenin pathway. Thus, the presence of mutations in LRP5 as well as Fzd-4 suggests the type and severity of disease or other phenotype by differential activation of signaling pathways and downstream phenotypic effects.

Detection of mutations in the gene for Fzd-4, or other genes, is performed preferably by techniques known in the art. In one embodiment DNA sequencing reactions using polymerase chain reaction (PCR) are used to screen the Fzd-4 gene in a subject. Methods for nucleotide sequencing of genetic or other biological material are known in the art.

The Fzd-4 nucleic acid sequences are optionally amplified before being sequenced. The term "amplified" defines the process of making multiple copies of the nucleic acid from a single or lower copy number of nucleic acid sequence molecule. The amplification of nucleic acid sequences is carried out in vitro by biochemical processes known to those of skill in the art such as PCR or by cloning and expansion.

One process of in vitro amplification, which is used according to this invention, is the polymerase chain reaction (PCR) illustratively described in U.S. Pat. Nos. 4,683,202 and 4,683,195. The term "polymerase chain reaction" refers to a process for amplifying a DNA base sequence using a heat-stable DNA polymerase and two oligonucleotide primers, one complementary to the (+)-strand at one end of the sequence to be amplified and the other complementary to the (-)-strand at the other end. Because the newly synthesized DNA strands subsequently serve as additional templates for the same primer sequences, successive rounds of primer annealing, strand elongation, and dissociation produce rapid and highly specific amplification of the desired sequence. Many polymerase chain processes are known to those of skill in the art and are operable in the process of the invention. For example, DNA is subjected to 30 to 35 cycles of amplification in a thermocycler as follows: 95°C. for 30 sec, 52 to 60°C. for 1 min, and 72°C for 1 min, with a final extension step of 72°C for 5 min. For another example, DNA is subjected to 35 polymerase chain reaction cycles in a thermocycler at a denaturing temperature of 95°C for 30 sec, followed by varying annealing temperatures ranging from 54 to 58°C for 1 min, an extension step at 70°C for 1 min, with a final extension step at 70°C for 5 min. The parameters of PCR cycling times and number of steps are dependent on the primer pair, their melting temperature, and other considerations known to those of skill in the art. It is appreciated that optimizing PCR parameters for various primer and/or probe sets is well within the skill of the art and is performed as mere routine optimization.

The amplification agent for PCR is optionally any compound or system that will function to accomplish the synthesis of primer extension products, including enzymes. Suitable enzymes for this purpose include, for example, E. coli DNA polymerase I, Taq polymerase, Klenow fragment of E. coli DNA polymerase I, T4 DNA polymerase, AmpliTaq Gold DNA Polymerase from Applied Biosystems, other available DNA polymerases, reverse transcriptase (preferably iScript RNase H+ reverse transcriptase), ligase, and other enzymes, including heat-stable enzymes (i.e., those enzymes that perform primer extension after being subjected to temperatures sufficiently elevated to cause denaturation). In a preferred embodiment, the enzyme is hot-start iTaq DNA polymerase from Bio-rad (Hercules, Calif.). Materials including enzymes operable to amplify target genes are optionally contained in a kit such as the Herculase PCR master mix (Stratagene, La Jolla, Calif.). Suitable enzymes will facilitate combination of the nucleotides in the proper manner to form the primer extension products that are complementary to each mutant nucleotide strand. Generally, synthesis is initiated at the 3'-end of each primer and proceed in the 5'-direction along the template strand, until synthesis terminates, producing molecules of different lengths. There are optionally amplification agents, however, that initiate synthesis at the 5'-end and proceed in the other direction, using the same process as described above. In any event, the process of the invention is not to be limited to the embodiments of amplification described herein.

The primers for use in amplifying the nucleic acid sequences of Fzd-4 are optionally prepared using any suitable process, such as conventional phosphotriester and phosphodiester processes or automated embodiments thereof so long as the primers are capable of hybridizing to the nucleic acid sequences of interest. One process for synthesizing oligonucleotides on a modified solid support is described in U.S. Pat. No. 4,458,066. The exact length of primer will depend on many factors, including temperature, buffer, and nucleotide composition. The primer generally should prime the synthesis of extension products in the presence of the inducing agent for amplification.

Design of primers for specific amplification of the Fzd-4 gene is similarly performed by methods known in the art. Preferably, primers are designed to flank a mutational site of interest. Primers used according to the process of the invention are complementary to each strand of nucleotide sequence to be amplified. The term "complementary" means that the primers hybridize with their respective strands under conditions that allow the agent for polymerization to function. In other words, the primers that are complementary to the flanking sequences hybridize with the flanking sequences and permit amplification of the nucleotide sequence. Preferably, the 3' terminus of the primer that is extended is perfectly base paired with the complementary flanking strand. Preferably, probes (if present) possess nucleotide sequences complementary to one or more strands of the amplification product such as from Fzd-4. More preferably, the primers are complementary to genetic sequences of Fzd-4 as found in GenBank accession number NG_011752.1, or to the mRNA sequence for Fzd-4 found at GenBank accession no. NM_012193.2. Most preferably, primers contain the nucleotide sequences of SEQ ID No: 1. It is appreciated that the complement of the aforementioned primer and probe sequences are similarly suitable for use in the instant invention. It is further appreciated that oligonucleotide sequences that hybridize with the inventive primer and probes are also similarly suitable.

In one embodiment primer pairs listed in Table 1 are operable to amplify regions of the Fzd-4 gene for sequence analyses.

| Primer | Sequence (5' to 3") | Primer Length | Product Lengh |
|---|---|---|---|
| Fzd4 exon 1 *forward* | CTGCTACCCCGATGCTG | 18 | 396 |
| Fzd4 exon 1 *reverse* | GGATGATCAACTTGGCATGG | 20 | |
| Fzd4 exon 2a *forward* | ATTGCCTGGAAGCATTCAAC | 20 | 570 |
| Fzd4 exon 2a *reverse* | CGCTCAGGGTAGGAAAACCT | 20 | |
| Fzd4 exon 2b *forward* | CAGCCTGTGTTTCATCTCCA | 20 | 567 |
| Fzd4 exon 2b *reverse* | ATTTTGAACAAGGCCACCAA | 20 | |
| Fzd4 exon 2c *forward* | CTGGCTTGTGCTATGTTGGA | 20 | 515 |
| Fzd4 exon 2c *reverse* | AAGCATGGAGGCTGACTAGC | 20 | |

Standard considerations of primer designs are taken into account when designing primers such as melting temperature, primer length, GC content, propensity to form secondary structure, identity of end nucleotides, and others known in the art. It is appreciated that modifications of primers of SEQ ID NOs: 3-10 are similarly operable.

Those of ordinary skill in the art will know of various amplification processes that are also used to increase the copy number of target nucleic acid sequence. The nucleic acid sequences detected in the process of the invention are optionally further evaluated, detected, cloned, sequenced, and the like, either in solution or after binding to a solid support, by any process usually applied to the detection of a specific nucleic acid sequence such as another polymerase chain reaction, oligomer restriction (Saiki et al., BioTechnology 3:1008 1012 (1985)), allele-specific oligonucleotide (ASO) probe analysis (Conner et al., PNAS 80: 278 (1983)), oligonucleotide ligation assays (OLAs) (Landegren et al., Science 241:1077 (1988)), RNase Protection Assay and the like. Molecular techniques for DNA analysis have been reviewed (Landegren et al., Science 242:229 237 (1988)). Following DNA amplification, the reaction product is optionally detected by Southern blot analysis, with or without using radioactive probes. In such a process, for example, a small sample of DNA containing the nucleic acid sequence obtained from the tissue or subject is amplified, and analyzed via a Southern blotting technique. The use of non-radioactive probes or labels is facilitated by the high level of the amplified signal. In one embodiment of the invention, one nucleoside triphosphate is radioactively labeled, thereby allowing direct visualization of the amplification product by autoradiography. In another embodiment, amplification primers are fluorescently labeled and run through an electrophoresis system. Visualization of amplified products is by laser detection followed by computer assisted graphic display, without a radioactive signal.

Other methods of detecting amplified oligonucleotide illustratively include gel electrophoresis, mass spectrometry, liquid chromatography, fluorescence, luminescence, gel mobility shift assay, fluorescence resonance energy transfer, nucleotide sequencing, enzyme-linked immunoadsorbent assay, affinity chromatography, chromatography, immunoenzymatic methods (Ortiz, A and Ritter, E, Nucleic Acids Res., 1996; 24:3280-3281), streptavidin-conjugated enzymes, DNA branch migration (Lishanski, A, et al., Nucleic Acids Res., 2000; 28(9):e42), enzyme digestion (U.S. Pat. No. 5,580,730), colorimetric methods (Lee, K., Biotechnology Letters, 2003; 25:1739-1742), or combinations thereof.

In one embodiment real-time PCR (RT-PCR) is used to detect the presence or absence of a mutation in Fzd-4. Methods of RT-PCR are known in the art such as that used in the TaqMan system. Illustratively, a probe complementary to the mutation of interest is used to detect the presence or absence of a mutation. A probe is preferably hybridized to a region of DNA that has a target mutation. Preferably, a probe differentially hybridizes to a region with a mutation and is selective for that mutation. Illustratively, a probe will have greater hybridization with a site that contains a mutation than with the site in the absence of the mutation. Greater hybridization is illustratively determined by differences in melting temperature between complete hybridization and incomplete hybridization.

Optionally, other detection systems using RT-PCR are operable herein. Illustratively, allele specific PCR with blocking reagent (ASB-PCR) is used as a method of detection in the present invention. Methods of ASB-PCR are found in Morlan, J, et al., 2009; PLoS ONE 4(2): e4584, doi:10.1371/journal.pone.0004584. Other methods such as that described in U.S. Application Publication No: 2008/0213756, are similarly operable.

In an alternative method the sequence of Fzd-4 mRNA is detected. This process preferably uses reverse transcription PCR to synthesize cDNA from RNA collected from a subject. The sequence of the synthesized cDNA is then obtained by PCR and sequencing methods as known in the art or as described herein and determination of the presence or absence of a mutation is performed.

In a preferred embodiment a probe such as that used for RT-PCR is labeled. The term "labeled" with regard to the probe is intended to encompass direct labeling of the probe by coupling (i.e., physically linking) a detectable substance to the probe, as well as indirect labeling of the probe by reactivity with another reagent that is directly labeled. Examples of indirect labeling include detection of a probe using a fluorescently labeled antibody and end-labeling or centrally labeling of a DNA probe with biotin such that it is detected with fluorescently labeled streptavidin.

The detection processes of the invention are optionally used to detect RNA (particularly mRNA) or genomic nucleic acid in a sample in vitro as well as in vivo. For example, in vitro techniques for detection of nucleic acid include northern hybridizations, in situ hybridizations, RT-PCR, reverse transcription PCR, and DNase protection. Furthermore, in vivo techniques illustratively include introducing into a subject organism a labeled antibody directed against a polypeptide or nucleic acid sequence of Fzd-4. Optionally, the antibody is labeled with a radioactive marker whose presence and location in the subject organism is detected by standard imaging techniques, including autoradiography.

The size of the primers used to amplify a portion of the nucleic acid sequence of Fzd-4 is optionally at least 5, and often 10, 15, 20, 25, or 30 nucleotides in length. Preferably, the GC ratio is above 30%, 35%, 40%, 45%, 50%, 55%, or 60% so as to prevent hair-pin structure on the primer. Furthermore, the amplicon is preferably of sufficient length to be detected by standard molecular biology methodologies. The forward primer is preferably shorter than the reverse primer. Techniques for modifying the Tₘ of either primer are operable herein. An illustrative forward or reverse primer contains LNA-dA and LNA-dT (Glen Research Corporation) so as to match Tₘ with a corresponding alternate primer.

An inventive process uses a polymerization reaction which employs a nucleic acid polymerizing enzyme, illustratively a DNA polymerase, RNA polymerase, reverse transcriptase, or mixtures thereof. It is further appreciated that accessory proteins or molecules are present to form the replication machinery. In a preferred embodiment the polymerizing enzyme is a thermostable polymerase or thermodegradable polymerase. Use of thermostable polymerases is well known in the art such as Taq polymerase available from Invitrogen Corporation (Carlsbad, Calif.). Thermostable polymerases allow a polymerization reaction to be initiated or shut down by changing the temperature or other condition in the reaction mixture without destroying activity of the polymerase.

Accuracy of the base pairing in the preferred embodiment of DNA sequencing is provided by the specificity of the enzyme. Error rates for Taq polymerase tend to be false base incorporation of 10⁻⁵ or less. (Johnson, Annual Reviews of Biochemistry, 1993: 62:685-713; Kunkel, Journal of Biological Chemistry, 1992; 267:18251-18254). Specific examples of thermostable polymerases illustratively include those isolated from Thermus aquaticus, Thermus thermophilus, Pyrococcus woesei, Pyrococcus furiosus, Thermococcus litoralis and Thermotoga maritima. Thermodegradable polymerases illustratively include E. coli DNA polymerase, the Klenow fragment of E. coli DNA polymerase, T4 DNA polymerase, T7 DNA polymerase and other examples known in the art. It is recognized in the art that other polymerizing enzymes are similarly suitable illustratively including E. coli, T7, T3, SP6 RNA polymerases and AMV, M-MLV, and HIV reverse transcriptases.

The polymerases are optionally bound to the primer. When the genetic material of Fzd-4 is a single-stranded DNA molecule illustratively due to heat denaturing, the polymerase is bound at the primed end of the single-stranded nucleic acid at an origin of replication. A binding site for a suitable polymerase is optionally created by an accessory protein or by any primed single-stranded nucleic acid.

In a further embodiment detection of PCR products is achieved by mass spectrometry. Mass spectrometry has several advantages over real-time PCR systems in that it is used to simultaneously detect the presence of Fzd-4 and decipher mutations in target nucleic acid sequences allowing identification of single or multiple mutations simultaneously. Further, mass spectrometers are prevalent in the clinical laboratory. Similar to fluorescence based detection systems, mass spectrometry is capable of simultaneously detecting multiple amplification products for a multiplexed and controlled approach to accurately quantifying components of biological or environmental samples.

Many mass spectrometry platforms are suitable for use in the instant invention illustratively including matrix assisted laser desorption ionization time of flight mass spectrometry (MALDI), electro spray mass spectrometry, electrospray ionization-Fourier transform ion cyclotron resonance mass spectrometry (ESI-FTICR), multi-stage mass spectrometry fragmentation analysis (MS/MS), mass spectrometry coupled with liquid chromatography such as high performance liquid chromatography mass spectrometry (HPLC) and ultra performance liquid chromatography isotope dilution tandem mass spectrometry (HPLC-ID/MS/MS), and variations thereof.

It is appreciated that numerous other detection processes are similarly suitable for measuring an amplification product by detecting a detection signal. Illustrative examples include, but are not limited to, liquid chromatography, mass spectrometry, liquid chromatography/mass spectrometry, static fluorescence, dynamic fluorescence, high performance liquid chromatography, ultra-high performance liquid chromatography, enzyme-linked immunoadsorbent assay, real-time PCR, gel electrophoresis, or combinations thereof.

The disclosed process optionally employs methods to regulate the expression, copy number, activity, or ligand affinity of Fzd-4. These processes are optionally performed by introducing a genetic sequence for Fzd-4 into a cell. The cell is optionally within a subject. In one embodiment the nucleotide sequence of SEQ ID NO: 1 is introduced into a cell. Preferably, the oligonucleotide sequence is a portion of an expression vector. It is appreciated that an oligonucleotide sequence encoding the full length Fzd-4 protein is not required in the subject invention. Any active portion thereof is similarly operable.

Thus, in an aspect the invention relates to an Fzd-4 compound for use as a medicament in the treatment of familial exudative vitreoretinopathy (FEVR) in a subject having a pathological condition of the retina or at risk of developing a pathological condition of the retina resulting from substitution mutation at nucleotide 349 and/or 542 of the nucleotide sequence genome encoding Fzd-4 polypeptide;
wherein the substitution mutation at nucleotide 349 and/or 542 of SEQ ID NO: 1 cause C117R and/or C181Y mutation in the Fzd-4 polypeptide

As used herein, the term "cell" illustratively includes a somatic cell, a germ cell, a progenitor cell, a cultured cell, a stem cell, a transfected cell, or combinations thereof.

As used herein, the term "administering" illustratively includes delivery of a molecule or a cell to a subject by a route illustratively including systemic administration, local administration, intravitreal injection, subconjuctival injection, sub-tenon injection, retrobulbar injection, suprachoroidal injection, surgical implantation, topical administration, iontophoretic delivery, oral, rectal, parenteral, intravenous, intramuscular, subcutaneous, intracisternal, intravaginal, intraperitoneal, intravesical, intraventricular, intracranial, intratumoral, local, transdermal, intrabuccal, intranasal, intrathecal, modifications thereof, or combinations thereof.

As used herein a compound is illustratively a protein, DNA, RNA, lipid, steroid, growth factor, antibody, antibody fragment, Fab', F(ab)₂, Fabc, Fv fragment, organic molecule, a cell, fragments thereof, mutations thereof, or mimics thereof. A compound is optionally recombinant. Most preferably, a compound encodes Fzd-4 such as a Fzd-4 compound.

As used herein, the term "stem cell" refers to a cell possessing self-replicating potential and the ability to give rise to terminally differentiated cells of single or multiple lineages. Stem cells are capable of generating identical progeny through unlimited numbers of cell divisions while retaining the ability to respond to physiological demands by producing daughters committed to differentiate.

Antibodies useful in the present systems include antibody fragments, such as Fab', F(ab) _{2,} Fabc, and Fv fragments. Antibody fragments are optionally produced by modification of whole antibodies or synthesized de novo using recombinant DNA methodologies, and further include "humanized" antibodies made by conventional or nonconventional techniques.

A compound administered herein is optionally supplemented with one or more agents effective in reducing inflammation, reducing pain, reducing or preventing tumor growth, reducing intraocular pressure, protecting cells, such as retinal neurons, reducing excitotoxicity, reducing infection, and reducing hemorrhage. A co-administered agent is optionally cytotoxic depending on the condition being treated. In addition, a co-administered agent optionally is a neurotoxic macromolecule, such as a botulinum neurotoxin, in combination with the non-neurotoxic macromolecule. A co-administered agent optionally is a small chemical compound in combination with the present macromolecules. Examples of chemical compounds operable herein illustratively include a small chemical compound, such as anecortave acetate, ketorlac tromethamine (such as Acular), gatifloxacin, ofloxacin, epinastine, and the like.

Optionally, a compound is delivered to a subject conjugated to a magnetic particle as described in U.S. Patent Application Publication 2004/0086572.

A Fzd-4 compound is illustratively a nucleotide sequence encoding a Fzd-4 protein, a Fzd-4 protein sequence, a fragment thereof, or a mimetic thereof. A Fzd-4 compound is illustratively SEQ ID NO: 1. A Fzd-4 compound is optionally purified.

A compound optionally is a mutant form of Fzd-4. It is appreciated that mutation of the conserved amino acid at any particular site is preferably mutatated to glycine or alanine. It is further appreciated that mutation to any neutrally charged, charged, hydrophobic, hydrophilic, synthetic, non-natural, non-human, or other amino acid is similarly operable.

Modifications and changes are optionally made in the structure (primary, secondary, or tertiary) of the Fzd-4 protein which are encompassed within the inventive compound that optionally result in a molecule having similar characteristics to the exemplary polypeptides disclosed herein. It is appreciated that changes in conserved amino acid bases are most likely to impact the activity of the resultant protein. However, it is further appreciated that changes in amino acids operable for receptor interaction, resistance or promotion of protein degradation, intracellular or extracellular trafficking, secretion, protein-protein interaction, post-translational modification such as glycosylation, phosphorylation, sulfation, and the like, optionally result in increased or decreased activity of an inventive compound while retaining some ability to alter or maintain a physiological activity. Certain amino acid substitutions for other amino acids in a sequence are known to occur without appreciable loss of activity.

In making such changes, the hydropathic index of amino acids are considered. According to the present invention, certain amino acids are substituted for other amino acids having a similar hydropathic index and still result in a polypeptide with similar biological activity. Each amino acid is assigned a hydropathic index on the basis of its hydrophobicity and charge characteristics. Those indices are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

Without intending to be limited to a particular theory, it is believed that the relative hydropathic character of the amino acid determines the secondary structure of the resultant polypeptide, which in turn defines the interaction of the polypeptide with other molecules. It is known in the art that an amino acid is substituted by another amino acid having a similar hydropathic index and still obtain a functionally equivalent polypeptide. In such changes, the substitution of amino acids whose hydropathic indices are within ±2 is preferred, those within ±1 are particularly preferred, and those within ±0.5 are even more particularly preferred.

As outlined above, amino acid substitutions are generally based on the relative similarity of the amino acid side-chain substituents, for example, their hydrophobicity, hydrophilicity, charge, size, and the like. Exemplary substitutions that take the foregoing characteristics into consideration are well known to those of skill in the art and include (original residue: exemplary substitution): (Ala: Gly, Ser), (Arg: Lys), (Asn: Gin, His), (Asp: Glu, Cys, Ser), (Gln: Asn), (Glu: Asp), (Gly: Ala), (His: Asn, Gln), (Ile: Leu, Val), (Leu: Ile, Val), (Lys: Arg), (Met: Leu, Tyr), (Ser: Thr), (Thr: Ser), (Tip: Tyr), (Tyr: Trp, Phe), and (Val: Ile, Leu). Embodiments of this disclosure thus contemplate functional or biological equivalents of a polypeptide as set forth above. In particular, embodiments of the polypeptides include variants having about 50%, 60%, 70%, 80%, 90%, and 95% sequence identity to the polypeptide of interest.

Multiple physiological activities are altered or maintained in the present inventive process. Physiological activities illustratively include vascularization, cell proliferation, cellular interaction, neuroprotection, growth, vascular regression, b-wave response, substantial oscillatory potential, or combinations thereof.

In a preferred disclosure Fzd-4 is administered to a subject as a factor suitable for expression within the eye or within a cell located in the eye. An exemplary system for the expression of protein is described in U.S. Patent Application Publication 2003/0129164, with particularity for disclosure of diseases, gene delivery methods, and gene expression methods. Any of a variety of vectors adapted for expression of Fzd-4 in a cell of the eye, preferably within a retinal cell, are also described in the present disclosure. Gene delivery vectors are optionally viral (e.g., derived from or containing sequences of viral DNA or RNA, preferably packaged within a viral particle), or non-viral (e.g., not packaged within a viral particle, including "naked" polynucleotides, nucleic acid associated with a carrier particle such as a liposome or targeting molecule, and the like).

The viral factor operable herein is illustratively a recombinant adeno-associated viral (rAAV) vector. It is appreciated the other suitable vectors known in the art are similarly operative. Additional illustrative vectors optionally include adenoviral vectors, alphaviral vectors, viruses illustratively including pox viruses illustratively canary pox virus or vaccinia virus, SV40, influenza virus, HIV, herpes, measles, Semliki Forest Virus, and coronavirus, as well as other viral systems. In addition, viral carriers are optionally homologous, non-pathogenic (defective), replication competent virus.

A particularly preferred gene delivery vector is a rAAV vector. A variety of rAAV vectors are optionally utilized to direct the expression of Fzd-4. An operable rAAV is generally, in order of 5' to 3', a 5' adeno-associated virus inverted terminal repeat, a coding sequence for the desired gene product (e.g., Norrin) operatively linked to a sequence which regulates its expression in a cell (e.g., a promoter sequence), and a 3' adeno-associated virus inverted terminal repeat. A promoter sequence is preferably a cell specific promoter sequence. In addition, the rAAV vector preferably has a polyadenylation sequence. The promoter is illustratively a constitutive promoter, a cell specific promoter, a selective molecule responsive promoter, or combinations thereof. Preferably a promoter is a cell specific promoter. More preferably, a promoter is a constitutive promoter that is cell type specific. Promoter sequences operative herein illustratively include the GFAP promoter, a retinal pigment cell specific promoter, a Muller cell specific promoter, promoters described in WO 2000/015822, a Cdc6 promoter, human ICAM-2 promoter, promoters and vectors described by Dai, C, et al., J Virology, 2004; 78:6209-6221, or other promoter known in the art.

Fzd-4 is optionally delivered to the eye by one or multiple routes illustratively including intraocularly, by topical application to the eye or by intraocular injection illustratively into the vitreous or subretinal (interphotoreceptor) space. Alternatively, delivery is local by insertion or injection into the tissue surrounding the eye, systemically through an oral route, or by subcutaneous, intravenous or intramuscular injection. Alternatively, delivery is by a catheter or by means of an implant, wherein such an implant is made of a porous, non-porous or gelatinous material, including membranes such as silastic membranes or fibers, biodegradable polymers, or proteinaceous material. A compound is illustratively administered prior to the onset of a pathological condition to prevent its occurrence, for example, during or prior to surgery on the eye, or immediately after the onset of the pathological condition or during the occurrence of an acute or protracted disease.

In a preferred disclosure, a compound is administered to the eye, preferably intraocularly to a variety of locations within the eye depending on the type of disease to be treated, prevented, or inhibited, and the extent of disease. Examples of suitable locations include the retina (e.g., for retinal diseases), the vitreous, or other locations in or adjacent the retina or in or adjacent to the eye.

In a preferred embodiment a cell is transfected with a nucleotide sequence encoding a Fzd-4 protein, a biologically active fragment of a Fzd-4 protein, or a Fzd-4 mimetic. Preferably, a Fzd-4 protein has between 5 and 500% the signaling activity or ligand binding affinity of wild-type Fzd-4 in either the presence or absence of a co-receptor. Preferably, a nucleotide sequence is cloned into an expression vector with a promoter. The promoter is illustratively a constitutive promoter, strong promoter (e.g., CMV promoters), inducible promoter, tissue-specific promoter, a cell specific promoter, a selective molecule responsive promoter, or combinations thereof.
Preferably a promoter is a cell specific promoter. More preferably, a promoter is a constitutive promoter that is cell type specific. Promoter sequences operative herein illustratively include the GFAP promoter, a retinal pigment cell specific promoter, a Muller cell specific promoter, promoters described in WO 2000/015822, a Cdc6 promoter, human ICAM-2 promoter, promoters and vectors described by Dai, C, et al., J Virology, 2004; 78:6209-6221, or other promoter known in the art. In a preferred embodiment a tetracycline responsive promoter is used to limit expression to instances when tetracycline is present in the system.

Numerous enhancers are operable to stimulate expression of Fzd-4 in a transfected cell. Illustratively an endothelial enhancer such as that described by Shaw, L C, et al., Gene Therapy, 2006; 13:752-760 is operative herein.

A cell is preferably a mammalian cell. A cell is optionally an endothelial cell, vascular cell, stem cell, immortalized cell, or combinations thereof. Preferably, a cell is a stem cell. It is appreciated that a stem cell illustratively includes a primary stem cells and lineage cells.

In a preferred part of the diclosure, the cell is a stem cell. A stem cell operable herein and its method of isolation and preparation is illustratively described in U.S. Patent Application Publication 2007/0154465. Stem cells are illustratively transfected with a vector encoding and inventive compound. Suitable vectors and methods of administration to a stem cell are also illustrated in U.S. Patent Application Publication 2007/0154465. Stem cells are optionally transiently transfected or transfected with a vector that provides incorporation into the host genome and constitutive expression.

In a preferred part of the disclosure, a cell is transfected with an expression vector encoding a Fzd-4 protein, a biologically active peptide fragment of a Fzd-4 protein, or a Fzd-4 mimetic and the transfected cell is administered to a subject. An advantage to this part of the disclosure is that possible barriers to optimal protein expression from host cells are overcome by capitalizing on known techniques of in vitro cell transfection. As such, delivery of Fzd-4 protein to the retina of a subject is greatly simplified and expression is improved or maximized. Further, when an endothelial cell, stem cell capable of differentiating into an endothelial cell, or other suitable cell type is used as the transfected cell, its subsequent administration to the eye of a subject provides suitable endogenous growth mediators such that cell survival is optimal and duration of successful delivery of a compound is maximized in concentration and time.

Optionally, a cell is derived from a patient. Illustratively, a stem cell is obtained from the patient, transfected, and administered to the patient as a Fzd-4 expressing cell. In a preferred part of the disclosure a retinal cell is obtained from a subject, is transfected with an expression vector expressing an inventive compound, and the transfected cell is administered to the subject.

Administration to the ocular environment in a subject is most preferably by injection into the vitreous. While patients who suffer from a pathological condition of the retina are amenable to multiple injections over the course of time, the use of transfected cells expressing a compound will preferably reduce the number of injections a subject must endure. Illustratively, a single injection of endothelial cells that have been transfected with a vector expressing an inventive compound onto the retina of a subject provides a long-term delivery of the inventive compound to the subject's retina. As such, a single therapeutic delivery will produce a clinical benefit.

In a most preferred embodiment the instant inventive method is employed with a subject that has a pathological condition. Pathological conditions are preferably vitreoretinopathy, retinopathy of prematurity, familial exudative vitreoretinopathy, Norrie disease, persistent fetal vasculature syndrome, Coats disease, macular degeneration, macular dystrophy, inherited dystrophy, cancer, growth of abnormal cells, osteoporosis-pseudoglioma syndrome, and other diseases of ocular vascular and retinal development.

As Fzd-4 protein has been implicated in forms of reproductive infertility, an assay for Fzd-4 concentration and/or activity represents a clinically significant value in treatment. Hess, A P, et al., Biol Reprod2007; 76:102-117; Hsieh, M, et al., Biol Reprod 2005; 73:1135-1146.

Fzd-4 is also implicated in human ovarian tumorigenesis through aberrant Wnt expression. Ricken, A, et al., Endocrinology 2002; 143:2741-2749. An assay for Fzd-4 concentration and/or activity represents a clinically significant value in treatment.

Preferably, a pathological condition results from, is caused by, is related to, or otherwise is associated with a mutation in the Fzd-4 gene, alteration in transcription to mRNA, mRNA survival, mRNA degradation, alternative mRNA splicing, protein translation, protein survival, protein degradation, protein trafficking, expression of protein on a cell membrane, protein secretion, or other abnormality in production of a functional Fzd-4 protein. This includes decreased production of Fzd-4 secondary to alternate gene expression or suppressed gene activation, seen in mature tissues. Most preferably, a pathological condition is associated with a mutation in the Fzd-4 protein. In a most preferred embodiment a pathological condition is a pathological condition of the retina.

In a most preferred part of the disclosure a compound is administered to a subject with a pathological condition and the presence of the compound alters or maintains at least one physiological activity. A physiological activity is preferably observed in the retina of a subject. This embodiment has the greatest efficacy in patients suffering from diseases that manifest themselves during development and childhood such as FEVR, ROP, or Norrie disease. Further, patients illustratively presenting with age related macular degeneration or young patients presenting with early stage disease will benefit from maintenance of retinal vascularization, oxygenation, or visual acuity. The instant inventive process is operable to reduce the rate of visual or other vascular related degeneration relative to subjects not receiving treatment.

A function of the retina is illustratively vascularization, cell proliferation, cellular interaction, neuroprotection, growth, vascular regression, b-wave response, substantial oscillatory potential, Wnt signaling, Fzd-4 signaling, CAMKII autophosphorylation, PKC activation, protein kinase A activation, activation of the MAPK pathway, stimulation of gene transcription, activation of intracellular signaling pathways illustratively including the PI3K/Akt pathway, preventing nuclear translocation of c-Jun N-terminal protein (JNK), or regulating caspase activation, stimulation of marker signaling, inhibition of marker signaling, maintenance of marker signaling, or combinations thereof. In a preferred embodiment retinal vascularization is increased. Most preferably, retinal vascularization approaches that observed in subjects that do not present with a pathological condition of the retina. It is appreciated that other markers of proper vascularization are also a function of the retina. These illustratively include development or differentiation of neuronal cells, or activity of neuronal cells.

A marker is illustratively a component of a compound. Illustratively, a cell is transfected with an expression vector that encodes a marker as well as a vector that encodes a compound. The administration of a marker provides the advantage of monitoring the function of the inventive method in cells or in a subject to whom a compound is administered. Markers operable herein are illustratively green fluorescent protein, luciferase, and β -galactosidase. It is appreciated that other suitable markers known in the art are similarly operable. A marker is optionally radioactive, luminescent, biologically active, or otherwise amenable to detection by methods known in the art.

The inventive methods and materials are optionally used in conjunction with treatments for mutations or disease related to the Fzd-4 protein, gene, RNA or other Fzd-4 molecule. Optionally, diagnosis is achieved by identification of mutations in both Fzd-4 and Norrin. Methods, materials, mutations, and other considerations related to Norrin are operable as additional methods, materials, compounds, and the like to the subject invention. These are illustratively described in International Patent Application No. PCT/US09/37792.

Methods involving conventional biological techniques are described herein. Such techniques are generally known in the art and are described in detail in methodology treatises such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992.

Various aspects of the present invention are illustrated by the following non-limiting examples. The examples are for illustrative. While the examples are generally directed to mammalian tissue, specifically, analyses of murine tissue, a person having ordinary skill in the art recognizes that similar techniques and other techniques known in the art readily translate the examples to other organisms such as humans. Reagents illustrated herein are commonly cross reactive between mammalian species or alternative reagents with similar properties are commercially available, and a person of ordinary skill in the art readily understands where such reagents are obtained.

### Example 1

### Association of Fzd-4 with FEVR and ROP

Subjects are diagnosed with FEVR or ROP based upon fundus examination, family history and gestational age. Each participant provides a blood sample from which genomic DNA is isolated from the leucocytes using Purgene Genomic DNA Purification Kit (Qiagen, Valencia, Calif.). When possible, genomic DNA from the relatives of subjects expressing a Fzd4 mutation are tested for sequence variations.

Genomic DNA from each patient is amplified and sequenced. The coding sequence (CDS) and flanking splice sites of the Fzd-4 gene are amplified from 100 ng of genomic DNA using Herculase PCR master mix (Stratagene, LaJolla, Calif.). Four sets of primers are used for specific amplification of desired Fzd-4 regions as listed in Table 1. Amplification conditions for each primer pair are as follows: 1 cycle at 98°C for 1 min; 40 cycles of 30 sec at 98°C; 30 sec at 55°C; and 1 min at 72°C; with a final extension for 10 min at 72°C. Amplified DNA is cleaned using QIAquick Multiwell Purification Kit (Qiagen, Valencia, Calif.) and subjected to sequence analyses using the Beckman Dye Terminator Cycle Sequence Quick Start Kit (Beckman Coulter, Inc, Fullerton, Calif.) and a Beckman CEQ8000 DNA Autosequencer.

Coding region mutations are found in 13 of the 123 subjects in the study as listed in Table 2. Nine of the 63 (14.3%) AdFEVR patients have mutations in the CDS of the Fzd-4 gene. Of these four mutations are heterozygous in AdFEVR including the Cys117Arg, Cys181Tyr, Gln505X, and the double mutation Pro33Ser/Pro168Ser. Four of 60 ROP subjects have the double mutation Pro33Ser/Pro168Ser. None of the identified mutations are observed in all 87 control subjects.

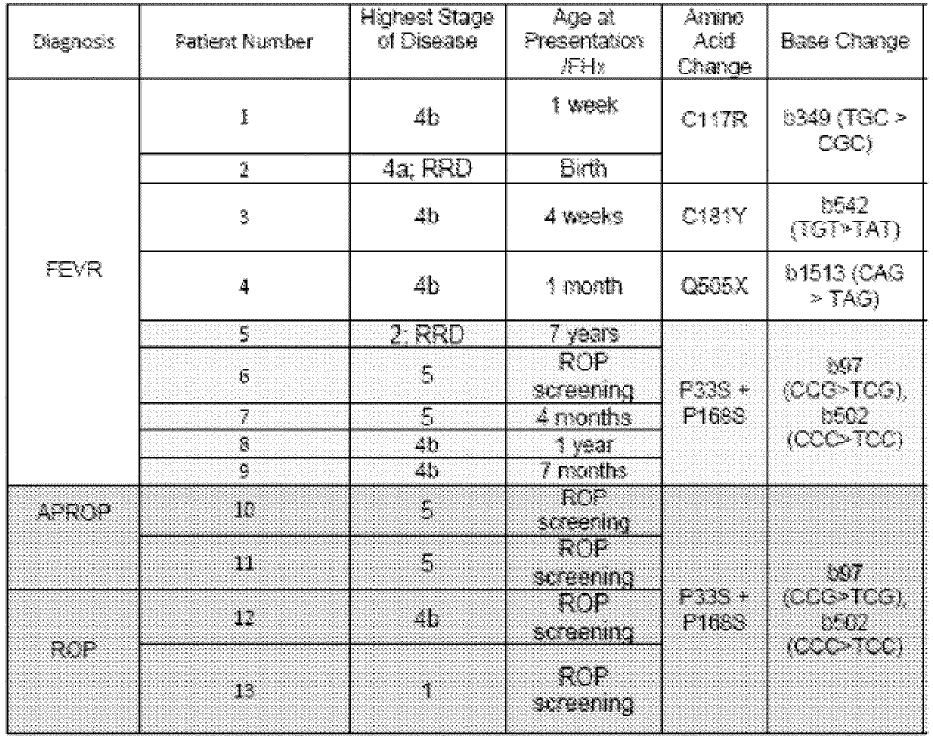

All subjects with a mutation have a retinal disease. The onset of disease varies within each mutation, but mutations Cys117Arg and Cys181Tyr each present either at birth or within one month thereafter and present with FEVR. No subjects with the single base pair mutation presents with either form of ROP. The double mutation Pro33Ser/Pro168Ser correlates with either FEVR or ROP.

### Example 2

### Establishment of Fzd-4 Expressing Cells

The coding region of Fzd-4 is amplified and cloned into a Gateway mammalian pDEST27 expression vector under the control of a CMV promoter using the Gateway system (Invitrogen, Carlsbad, Calif.) as per the manufacturer's protocols. The vector is transfected into E. coli for plasmid amplification. Cells are collected, lysed, and amplified plasmid is collected and isolated by standard techniques. Cells from the BGO1V human embryonic stem cell line are transfected with the Fzd-4 expressing plasmid by electroporation using the NEON transfection system (Invitrogen, Carlsbad, Calif.) as per the manufacturer's protocols. Briefly, cells are transfected with 0.5 µg of plasmid DNA at a pulse voltage of 1,200 volts with a pulse width of 30 ms one time. Cells successfully transfected are selected and expanded.

### Example 3

The presence of transfected Fzd-4 expression construct produces earlier development of vascular channels in developing stem cells.

Endothelial stem cells (EC cells) of either of Example 2 or in the absence of transfected overexpressed Fzd-4 are differentiated and analyzed essentially as described by Ng, Y, et al. Laboratory Investigation, 2004; 84:1209-1218. Briefly, trypsinized ES cells are suspended in culture medium including high-glucose Dulbecco's modified Eagle's medium (DMEM, GIBCO BRL, Grand Island, N.Y., USA) with 15% fetal bovine serum (Hyclone, Utah, USA), sodium pyruvate (GIBCO, stock solution diluted 1:100), nonessential amino acids (GIBCO, stock solution diluted 1:100), β-mercaptoethanol (GIBCO, final concentration 30 µM), 190 µg/ml of L-glutamine, 60 U/ml of penicillin G, 60 µg/ml of streptomycin (glutamine pen-strep mix, Irvine Scientific, Santa Ana, Calif., USA), supplemented with media (1:300 dilution) conditioned by Chinese hamster ovary cells overexpressing LIF (provided by Genetics Institute, Cambridge, Mass., USA) as a source of LIF to maintain the ES cells in an undifferentiated state in a humidified tissue culture incubator at 10% CO₂ and 37°C, and passaged every 2-3 days.

ES cells are differentiated into embroyid bodies. A total of 60 aliquots (30 µl) of ES cell suspension containing 2.5x10³ cells are plated as individual drops onto 100 mm² bacteriological dishes (Valmark Inc., Canada). The plates are then gently inverted into hanging drops and the cells incubated. The CEB are cultivated for 40-45 h, and then the dishes are turned right side up and flooded with 10 ml of ES culture media without LIF. The culture media is replaced every three days. The attached cultures of CEB are transferred at day 4 or day 5 to gelatin-coated tissue culture plates or gelatin-coated cover glasses, onto which the CEB attaches, flattens and spreads. Cells are fixed and stained for PECAM-1 expression as described by Ng, Y, et al.

PECAM-1 staining (red) reveals that in the presence of cells expressing transfected Fzd-4 demonstrate earlier vascular channel development than non-transfected control cells with vascular channels appearing at week 2 in Fzd-4 expressing groups and not readily apparent in untransfected until week 4 (FIG. 1).

### Example 4

Intravitreal injection of Fzd-4. The transfected cells of Example 2 are exposed to the retina by intravitreal injection substantially as described by Jiang, C, et al., Mol V is, 2007; 13:1783-92. Intravitreal injection is performed under general anesthesia using an ophthalmic operating microscope (Moller-Wedel GmbH, Wedel, Germany) using beveled glass micro-needles with an outer diameter of approximately 100 µm. A 30-gauge hypodermic needle is used to perforate the sclera 1.5 mm behind the limbus. Five µl of test sample is optionally injected by way of this passage into the vitreous using a 50 µl Hamilton Syringe (Hamilton Co, Reno, Nev.). To ensure adequate delivery and prevent shock the needle is held in place for one min after the injection is completed and subsequently withdrawn slowly. In addition, paracentesis is simultaneously performed to relieve pressure and thereby prevent reflux.

### Example 5

NDMA induced retinal damage is corrected by administration of cells expressing Fzd-4. Eighteen adult male ddY mice weighing 36 to 43 g each are anesthetized with 3.0% isoflurane and maintained with 1.5% isoflurane in 70% N₂O and 30% O₂. Body temperatures are maintained between 37.0°C and 37.5°C with the aid of a heating pad and a heating lamp. One eye of each animal is designated a control eye with the other eye used as a test eye. At day 0 all test eyes and 1/2 of control eyes are injected with n-methyl D-aspartate (NMDA) (Sigma-Aldrich) into the vitreous body at 200 nM per eye dissolved in 0.01 M phosphate-buffered saline (PBS) to induce retinal damage. The remaining control eyes are injected with an equal volume of PBS. At day 1, test eyes are injected with ES cells as prepared as in Example 2 or control untransfected ES cells. One drop of levofloxacin ophthalmic solution (Santen Pharmaceuticals Co. Ltd., Osaka, Japan) is applied topically to each eye after intravitreous injection. Animals are returned to cages and maintained until day 6 at which time they are sacrificed and all eyes are enucleated for retinal tissue fixation and immunohistochemistry. Eyes are enucleated, fixed in 4% paraformaldehyde overnight at 4°C, immersed in 20% sucrose for 48 hours at 4°C, and embedded in optimum cutting temperature (OCT) compound (Sakura Finetechnical Co., Ltd., Tokyo, Japan). Transverse, 10 µm thick cryostat sections are cut and placed onto slides (Mas Coat). Sections are subsequently processed for immunocytochemistry following staining for Glutamine synthatase and staining for DNA using DAPI.

NMDA treated eyes develop numerous lesions indicative of neuronal damage. Treatment with Fzd-4 expressing ES cells illustrates much less retinal damage. Treatment of mouse eyes with ES cells expressing Fzd-4 is protective of damage as illustrated by nearly complete protection against NMDA damage as compared to control eyes.

### Example 6

Treatment of FEVR diseased eyes with cells expressing Fzd-4 promotes improved retinal response. Subjects who have been diagnosed with FEVR or are at risk for FEVR are exposed to Fzd-4 prepared essentially as described in Example 2 and administered as described in Example 3. Administration is performed either a single time, or repeated weekly depending on the delivery mechanism used. At weeks 1, 2, 4, and 20 subjects are analyzed for improved retinal physiological activity using electroretinograms by methods known in the art or illustratively as described by Wu, W, et al., Molecular Vision, 2004; 10:93-102. Subjects treated with cells expressing Fzd-4 demonstrate improved a- and b-wave measurements at all time points tested.

Various modifications of the present invention, in addition to those shown and described herein, will be apparent to those skilled in the art of the above description.

Patents and publications mentioned in the specification are indicative of the levels of those skilled in the art to which the invention pertains.

The foregoing description is illustrative of particular embodiments of the invention, but is not meant to be a limitation upon the practice thereof. The following claims, including all equivalents thereof, are intended to define the scope of the invention.

### REFERENCE SEQUENCES

Fzd-4 Nucleotide Sequence (SEQ ID NO: 1) 
5'-ATGGCCTGGCGGGGCGCAGGGCCGAGCGTCCCGGGGGCGCCCGGGGGCGTCGGT
CTCAGTCTGGGGTTGCTCCTGCAGTTGCTGCTGCTCCTGGGGCCGGCGCGGGGCTTC
GGGGACGAGGAAGAGCGGCGCTGCGACCCCATCCGCATCTCCTGTGCCAGAACCTC
GGCTACAACGTGACCAAGATGCCCAACCTGGTTGGGCACGAGCTGCAGACGGACGC
CGAGCTGCAGCTGACAACTTTCACACCGCTCATCCAGTACGGCTGCTCCAGCCAGCT
GCAGTTCTTCCTTTGTTCTGTTTATGTGCCAATGTGCACAGAGAAGATCAACATCCC
CATTGGCCCATGCGGCGGCATGTGTCTTTCAGTCAAGAGACGCTGTGAACCCGTCCT
GAAGGAATTTGGATTTGCCTGGCCAGAGAGTCTGAACTGCAGCAAATTCCCACCAC
AGAACGACCACAACCACATGTGCATGGAAGGGCCAGGTGATGAAGAGGTGCCCTT
ACCTCACAAAACCCCCATCCAGCCTGGGGAAGAGTGTCACTCTGTGGGAACCAATT
CTGATCAGTACATCTGGGTGAAAAGGAGCCTGAACTGTGTGCTCAAGTGTGGCTAT
GATGCTGGCTTATACAGCCGCTCAGCCAAGGAGTTCACTGATATCTGGATGGCTGT
GTGGGCCAGCCTGTGTTTCATCTCCACTGCCTTCACAGTACTGACCTTCCTGATCGA
TTCTTCTAGGTTTTCCTACCCTGAGCGCCCCATCATATTTCTCAGTATGTGCTATAAT
ATTTATAGCATTGCTTATATTGTCAGGCTGACTGTAGGCCGGGAAAGGATATCCTGT
GATTTTGAAGAGGCAGCAGAACCTGTTCTCATCCAAGAAGGACTTAAGAACACAGG
ATGTGCAATAATTTTCTTGCTGATGTACTTTTTTGGAATGGCCAGCTCCATTTGGTGG
GTTATTCTGACACTCACTTGGTTTTTGGCAGCAGGACTCAAATGGGGTCATGAAGCC
ATTGAAATGCACAGCTCTTATTTCCACATTGCAGCCTGGGCCATCCCCGCAGTGAAA
ACCATTGTCATCTTGATTATGAGACTGGTGGATGCAGATGAACTGACTGGCTTGTGC
TATGTTGGAAACCAAAATCTCGATGCCCTCACCGGGTTCGTGGTGGCTCCCCTCTTT
ACTTATTTGGTCATTGGAACTTTGTTCATTGCTGCAGGTTTGGTGGCCTTGTTCAAAA
TTCGGTCAAATCTTCAAAAGGATGGGACAAAGACAGACAAGTTAGAAAGACTGAT
GGTCAAGATTGGGGTGTTCTCAGTACTGTACACAGTTCCTGCAACGTGTGTGATTGC
CTGTTATTTTTATGAAATCTCCAACTGGGCACTTTTTCGGTATTCTGCAGATGATTCC
AACATGGCTGTTGAAATGTTGAAAATTTTTATGTCTTTGTTGGTGGGCATCACTTCA
GGCATGTGGATTTGGTCTGCCAAAACTCTTCACACGTGGCAGAAGTGTTCCAACAG
ATTGGTGAATTCTGGAAAGGTAAAGAGAGAGAAGAGAGGAAATGGTTGGGTGAAG
CCTGGAAAAGGCAGTGAGACTGTGGTATAA-3'
**Fzd-4 Protein Sequence (SEQ ID NO: 2)**

## Claims

1. A process for diagnosing a disease or a susceptibility to a disease related to expression, conformation, or activity of the Transmembrane receptor frizzled-4 (Fzd-4) polypeptide in a subject comprising:
- providing a sample from said subject;
- determining in the sample the presence or absence of a substitution mutation in the nucleotide sequence encoding Fzd-4 in the genome at nucleotide 349 and/or 542 of SEQ ID NO: 1; and
- diagnosing the presence or absence of said disease or susceptibility to said disease in said subject or descendents of said subject;
wherein presence of the substitution mutation in the nucleotide sequence encoding Fzd-4 in the genome at nucleotide 349 and/or 542 of SEQ ID NO: 1 indicates the presence or susceptibility for a disease related to expression, conformation, or activity of the Fzd-4 polypeptide in the subject;
wherein the substitution mutation at nucleotide 349 and/or 542 of SEQ ID NO: 1 cause C117R and/or C181Y mutation in the Fzd-4 polypeptide;
wherein the disease is familial exudative vitreoretinopathy (FEVR).

2. The method of claim 1, wherein mutations at nucleotides 349 and 542 of SEQ ID NO 1 are present and the FEVR, is a FEVR with onset at birth or within one month thereafter.

3. An Fzd-4 for use as a medicament in the treatment of familial exudative vitreoretinopathy (FEVR) in a subject having a pathological condition of the retina or at risk of developing a pathological condition of the retina resulting from substitution mutation at nucleotide 349 and/or 542 of the nucleotide sequence genome encoding Fzd-4 polypeptide;
wherein the substitution mutation at nucleotide 349 and/or 542 of SEQ ID NO: 1 cause C117R and/or C181Y mutation in the Fzd-4 polypeptide

4. The Fzd-4 for use according to claim 3, wherein the Fzd-4 is nucleotide sequence encoding an Fzd-4 protein, an Fzd-4 protein sequence, a fragment thereof, or a mimetic thereof.

5. The Fzd-4 for use according to of any of claims 3-4, wherein said medicament is for administration by one of: systemic administration, local administration, injection, topical administration, intraocular, or iontophoretic delivery.

6. The Fzd-4 for use according to of any of claims 3-5, wherein said Fzd-4 is recombinant.

7. The Fzd-4 for use according to of any of claims 3-6, wherein said Fzd-4 comprises the nucleotide sequence of SEQ ID NO: 1.

8. The Fzd-4 for use according to of any of claims 3-7, wherein said Fzd-4 encodes the polypeptide sequence of SEQ ID NO: 2.

9. The Fzd-4 for use according to any of claims 3-8, wherein said compound has from about 5 to 99 % the biological activity of the Fzd-4 polypeptide of SEQ ID NO: 2.

10. The process of claim 1, wherein the step of determining in the sample the presence or absence of a substitution mutation in the nucleotide sequence encoding Fzd-4 represented by SEQ ID NO: 1, further comprises determining the presence of mutation at nucleotide 97, and/or 502, and/or 1513 of the nucleotide sequence represented by SEQ ID NO: 1.

## Patentansprüche

1. Verfahren zum Diagnostizieren einer Erkrankung oder einer Anfälligkeit für eine Erkrankung in Verbindung mit Expression, Konformation oder Aktivität des Transmembranrezeptor-Polypeptids Frizzled-4 (Fzd-4) bei einem Subjekt, umfassend:
- Bereitstellen einer Probe von dem Subjekt;
- Bestimmen, in der Probe, des Vorhandenseins oder der Abwesenheit einer Substitutionsmutation in der Nukleotidsequenz, die Fzd-4 in dem Genom an Nukleotid 349 und/oder 542 von SEQ ID NO: 1 kodiert; und
- Diagnostizieren des Vorhandenseins oder der Abwesenheit von der Erkrankung oder Anfälligkeit für die Erkrankung bei dem Subjekt oder Nachkommen des Subjekts;
wobei Vorhandensein der Substitutionsmutation in der Nukleotidsequenz, die Fzd-4 in dem Genom an Nukleotid 349 und/oder 542 von SEQ ID NO: 1 kodiert, das Vorhandensein oder die Anfälligkeit für eine Erkrankung in Verbindung mit Expression, Konformation oder Aktivität des Fzd-4-Polypeptids in dem Subjekt anzeigt;
wobei die Substitutionsmutation an Nukleotid 349 und/oder 542 von SEQ ID NO: 1 C117R- und/oder C181Y-Mutation in dem Fzd-4-Polypeptid verursacht;
wobei die Erkrankung familiäre exsudative Vitreoretinopathie (FEVR) ist.

2. Verfahren nach Anspruch 1, wobei Mutationen an Nukleotiden 349 und 542 von SEQ ID NO 1 vorhanden sind und die FEVR eine FEVR mit Einsetzen bei Geburt oder innerhalb eines Monats danach ist.

3. Fzd-4 zur Anwendung als ein Medikament bei der Behandlung von familiärer exsudativer Vitreoretinopathie (FEVR) bei einem Subjekt mit einem pathologischen Zustand der Retina oder mit Risiko für Entwicklung eines pathologischen Zustands der Retina aufgrund von Substitutionsmutation an Nukleotid 349 und/oder 542 des Nukleotidsequenzgenoms, das das Fzd-4-Polypeptid kodiert;
wobei die Substitutionsmutation an Nukleotid 349 und/oder 542 von SEQ ID NO: 1 C117R- und/oder C181Y-Mutation in dem Fzd-4-Polypeptid verursacht.

4. Fzd-4 zur Anwendung gemäß Anspruch 3, wobei das Fzd-4 eine Nukleotidsequenz ist, die ein Fzd-4-Protein, eine Fzd-4-Proteinsequenz, ein Fragment davon oder ein Mimetikum davon kodiert.

5. Fzd-4 zur Anwendung nach einem der Ansprüche 3-4, wobei das Medikament zur Verabreichung durch eines der Folgenden bestimmt ist: systemische Verabreichung, lokale Verabreichung, Injektion, topische Verabreichung, intraokular oder iontophoretische Bereitstellung.

6. Fzd-4 zur Anwendung nach einem der Ansprüche 3-5, wobei das Fzd-4 rekombinant ist.

7. Fzd-4 zur Anwendung nach einem der Ansprüche 3-6, wobei das Fzd-4 die Nukleotidsequenz von SEQ ID NO: 1 umfasst.

8. Fzd-4 zur Anwendung nach einem der Ansprüche 3-7, wobei das Fzd-4 die Polypeptidsequenz von SEQ ID NO: 2 kodiert.

9. Fzd-4 zur Anwendung nach einem der Ansprüche 3-8, wobei die Verbindung von etwa 5 bis 99 % der biologischen Aktivität des Fzd-4-Polypeptids von SEQ ID NO: 2 hat.

10. Verfahren nach Anspruch 1, wobei der Schritt des Bestimmens, in der Probe, des Vorhandenseins oder der Abwesenheit einer Substitutionsmutation in der Nukleotidsequenz, die Fzd-4 kodiert, dargestellt durch SEQ ID NO: 1, ferner Bestimmen des Vorhandenseins von Mutation an Nukleotid 97 und/oder 502 und/oder 1513 der Nukleotidsequenz, dargestellt durch SEQ ID NO: 1, umfasst.

## Revendications

1. Procédé de diagnostic d'une maladie ou de vulnérabilité à une maladie liée à l'expression, la conformation ou l'activité du polypeptide récepteur transmembranaire Frizzled-4 (Fzd-4) chez un sujet comprenant :
- la fourniture d'un échantillon obtenu dudit sujet ;
- la détermination dans l'échantillon de la présence ou de l'absence d'une mutation par substitution dans la séquence nucléotidique codant pour Fzd-4 dans le génome au nucléotide 349 et/ou 542 de SEQ ID NO: 1 ; et
- le diagnostic de la présence ou de l'absence de ladite maladie ou vulnérabilité à ladite maladie chez ledit sujet ou chez les descendants dudit sujet ;
la présence de la mutation par substitution dans la séquence nucléotidique codant pour Fzd-4 dans le génome au nucléotide 349 et/ou 542 de SEQ ID NO: 1 indiquant la présence ou la vulnérabilité à une maladie liée à l'expression, la conformation ou l'activité du polypeptide Fzd-4 chez le sujet ;
la mutation par substitution au nucléotide 349 et/ou 542 de SEQ ID NO: 1 causant la mutation C117R et/ou C181Y dans le polypeptide Fzd-4 ;
la maladie étant une vitréorétinopathie exsudative familiale (FEVR).

2. Procédé selon la revendication 1, dans lequel des mutations aux nucléotides 349 et 542 de SEQ ID NO 1 sont présentes et la FEVR est une FEVR se manifestant à la naissance ou dans le mois qui suit.

3. Fzd-4 pour utilisation comme médicament dans le traitement d'une vitréorétinopathie exsudative familiale (FEVR) chez un sujet présentant un état pathologique de la rétine ou un risque de développement d'un état pathologique de la rétine résultant de la mutation par substitution au nucléotide 349 et/ou 542 du génome de séquence nucléotidique codant pour le polypeptide Fzd-4 ;
la mutation par substitution au nucléotide 349 et/ou 542 de SEQ ID NO: 1 causant la mutation C117R et/ou C181Y dans le polypeptide Fzd-4.

4. Fzd-4 pour utilisation selon la revendication 3, dans lequel le Fzd-4 est une séquence nucléotidique codant pour une protéine Fzd-4, une séquence de la protéine Fzd-4, un fragment de celle-ci ou un agent mimétique de celle-ci.

5. Fzd-4 pour utilisation selon l'une quelconque des revendications 3-4, ledit médicament étant destiné à être administré par l'une des méthodes suivantes : administration systémique, administration locale, injection, administration topique, administration intraoculaire ou iontophorétique.

6. Fzd-4 pour utilisation selon l'une quelconque des revendications 3-5, ledit Fzd-4 étant recombinant.

7. Fzd-4 pour utilisation selon l'une quelconque des revendications 3-6, ledit Fzd-4 comprenant la séquence nucléotidique de SEQ ID NO: 1.

8. Fzd-4 pour utilisation selon l'une quelconque des revendications 3-7, ledit Fzd-4 codant pour la séquence polypeptide de SEQ ID NO: 2.

9. Fzd-4 pour utilisation selon l'une quelconque des revendications 3-8, ledit composé ayant autour de 5 à 99 % d'activité biologique du polypeptide Fzd-4 de SEQ ID NO: 2.

10. Procédé selon la revendication 1, dans lequel l'étape consistant à déterminer dans l'échantillon la présence ou l'absence d'une mutation par substitution dans la séquence nucléotidique codant pour Fzd-4 représenté par SEQ ID NO: 1, comprend en outre la détermination de la présence d'une mutation au nucléotide 97, et/ou 502, et/ou 1513 de la séquence nucléotidique représentée par SEQ ID NO: 1.
